# EUROPÄISCHE PATENTSCHRIFT

(11) **EP 3 199 106 B1**
(45) Veröffentlichungstag und Bekanntmachung des Hinweises auf die Patenterteilung: **09.09.2020**
(21) Anmeldenummer: 17168227.1
(22) Anmeldetag: 26.04.2017
(51) Int. Cl.: A61B 6/00, A61B 8/00, A61B 6/08, A61B 8/08

(54) **VORRICHTUNG UND VERFAHREN ZUR ULTRASCHALLUNTERSUCHUNG**
METHOD AND DEVICE FOR ULTRASOUND INSPECTION
PROCÉDÉ ET DISPOSITIF DESTINÉS À L'EXAMEN PAR ULTRASONS

(43) Veröffentlichungstag der Anmeldung: 02.08.2017
(73) Patentinhaber: Siemens Healthcare GmbH, 91052 Erlangen (DE)
(72) Erfinder: Fieselmann, Andreas, 91052 Erlangen (DE)

(56) Entgegenhaltungen:
- EP-A1- 2 853 200
- US-A1- 2003 167 004
- US-A1- 2014 180 082

## Beschreibung

Zur Ortung und Verifizierung von pathologischen Symptomen von beispielsweise einem Organ eines Patienten bedarf es oftmals unterschiedlicher bildgebender Verfahren um Bilder von einer Region eines Patienten anzulegen. Zur Erstdiagnose könnten beispielsweise Ultraschallaufnahmen sowie einzelne oder 3D-Röntgenaufnahmen erstellt werden. Von Nachteil bei den unterschiedlichen Aufnahmen ist, dass diese von zwei separaten Aufnahmesystemen erstellt werden. So werden neben steuerbaren und exakt ausrichtbaren Röntgenaufnahmeeinheiten einzelne oder eine Serie von Röntgenaufnahmen von zu untersuchenden Regionen bei einem Patienten angelegt und zu diesen ergänzend in einem nachfolgenden Arbeitsschritt Ultraschallaufnahmen mit einer manuell geführten bzw. auszurichtenden Ultraschalleinheit aufgenommen. Eine mögliche Zuordnung von Auffälligkeiten in dem Röntgen- bzw. dem Ultraschallbild liegt im Beurteilungsvermögen des behandelnden Arztes.
In der EP 2 853 200 A1 ist eine Vorrichtung zur Ausrichtung bzw. Positionierung eines diagnostischen Röntgen-Gerätes in Abhängigkeit von der Ausrichtung bzw. Positionierung eines diagnostischen Ultraschall-Gerätes mittels drei Winkelmessern bzw. Winkel-Sensoren offenbart.
In der US 2014/180082 A1 ist eine Vorrichtung zum synchronen Abtasten bzw. Scannen eines Untersuchungsobjektes mit einem Ultraschallwandler und einem Röntgendetektor offenbart.
In der US 2003/167004 A1 ist eine Vorrichtung offenbart, welche das Erfassen einer Ultraschallaufnahme und einer Röntgenaufnahme bei einer Mammographieuntersuchung an einem Gerät ermöglicht.
Der vorliegenden Erfindung liegt die Aufgabe zugrunde, eine Vorrichtung und ein Verfahren anzugeben, mit den zumindest zwei verschiedenen Bildaufnahmeverfahren möglich werden.

Die Erfindung wird durch die Merkmale der Patentansprüche 1 oder 8 gelöst.

Mit dem Gegenstand der Erfindung liegt eine Vorrichtung und ein dazugehöriges Verfahren vor, bei dem mit einer Röntgeneinheit Röntgenaufnahmen von einem Objekt erstellbar sind, wobei die Röntgeneinheit mit einer ausrichtbaren Positioniereinheit auf das Objekt ausrichtbar ist. Bei dieser Vorrichtung und dem dazugehörigen Verfahren ist eine Ultraschalleinheit zum Erstellen von Ultraschallaufnahmen vorgesehen, wobei die Ultraschalleinheit mit der Positioniereinheit der Röntgeneinheit auf das Objekt ausgerichtet wird.

Die Erfindung bringt den Vorteil mit sich, dass die Röntgen- und Ultraschallbilder mit einer Vorrichtung und einem dazugehörigem Verfahren angelegt werden, wobei den anzulegenden Bildern jeweils das gleiche Koordinatensystem zu Grunde gelegt wird.

Die Erfindung bringt den Vorteil mit sich, dass Merkmale im Röntgen- und Ultraschallbild zueinander in Bezug setzbar sind.

Die Erfindung bringt den Vorteil mit sich, dass die Ultraschall- wie auch die Röntgeneinheit als telemedizinische Einrichtung einsetzbar sind.

Die Erfindung bringt den Vorteil mit sich, dass die Kinematik der Positioniereinheit einer bestehenden Röntgeneinheit für die Ausrichtung bzw. Führung einer Ultraschalleinheit verwendet wird.

Die Erfindung bringt den Vorteil mit sich, dass die fernsteuerbare Positioniereinheit einen Arzt entlastet.
Die Erfindung bringt den Vorteil mit sich, dass Bilder mit unterschiedlichen Kontrasten anlegbar sind, wobei Weichteile im Ultraschallbild und Knochen im Röntgenbild gut sichtbar sind.

Nachfolgend wird der Gegenstand der Technik der Erfindung anhand von Zeichnungen näher erläutert.

Es zeigen:
- Figur 1: eine Vorderansicht einer Ultraschalleinheit mit Koppeleinheit,
- Figur 2: eine schematische Darstellung einer Gerätekombination,
- Figur 3: eine Röntgeneinheit,
- Figur 4: eine Ansicht auf eine Gerätekombination und
- Figur 5: eine weitere Ansicht auf die Gerätekombination.

Der Gegenstand der Erfindung bildet eine Vorrichtung und ein dazugehöriges Verfahren zur Röntgen- und/oder Ultraschalluntersuchung bei der eine Ultraschalleinheit mit einer Koppeleinheit an einer Röntgeneinheit befestigbar ist. Die Positionierung und Ausrichtung der Röntgeneinheit wird mit einer Positioniereinheit durchgeführt. In einer Ausführungsvariante weist die Positioniereinheit die zur Positionierung und Aussteuerung der Röntgeneinheit nötigen ansteuerbaren Achsen auf. Die Ultraschalleinheit wird mit der Positionierungseinheit der Röntgeneinheit am Patienten positioniert und entsprechend den an einem Bedienelement eingegebenen Steuerbefehle eines Assistenten oder eines Arztes geführt bzw. ausgerichtet. Zur uneingeschränkten Ausrichtung der Ultraschalleinheit weist die Positioniereinheit eine Vielzahl von Freiheitsgraden auf.

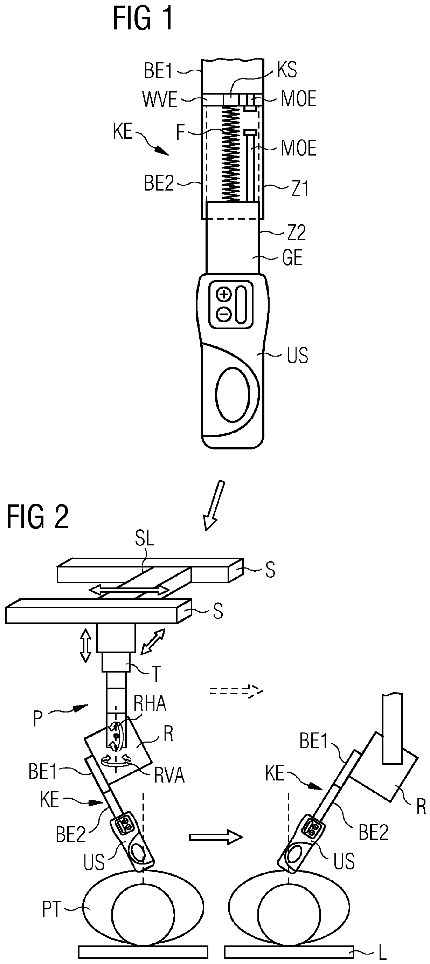

In Figur 1 ist eine Ultraschalleinheit US mit einer Koppeleinheit KE abgebildet. Die Koppeleinheit KE bildet sich in dieser Ausgestaltung und Darstellung aus einer ersten Befestigungseinheit BE1 und einer zweiten Befestigungseinheit BE2. Zwischen der ersten und der zweiten Befestigungseinheit BE1, BE2 ist eine Wirkverbindungselementeeinheit WVE angeordnet. Während die erste Befestigungseinheit BE1 befestigungsspezifische Elemente für eine Halterung oder speziell an der Röntgen- und/oder der Positionierungseinheit P vorgesehene Andockstellen aufweist, ist die zweite Befestigungseinheit BE2 derart ausgebildet, dass diese beispielsweise aus einem ersten und zweiten Hohlkörper Z1, Z2 gebildet ist. Der zweite Hohlkörper Z2 ist über Führungselemente in den ersten Hohlkörper Z1 integrierbar bzw. kann in diesen geschoben werden. Die Aufnahme des zweiten Hohlkörpers Z2 in den Innenraum des ersten Hohlzylinders Z1 wird über mindestens ein Dämpfungselement reguliert. Als Dämpfungselement ist in dieser Ausführungsvariante eine im ersten Hohlkörper Z1 angeordnete Feder F vorgesehen. An dem offenen Ende des zweiten Hohlkörpers Z2 ist die Ultraschalleinheit US angeordnet. Zwischen der ersten und zweiten Befestigungseinheit BE1, BE2 ist eine Wirkverbindungselementeeinheit WVE ausgebildet, die eine toleranzfreie Verbindung zwischen beiden Befestigungseinheiten BE1, BE2 ermöglicht. Die Einführung des zweiten Hohlkörpers Z2 in den ersten Hohlkörper Z1 wird durch eine im Inneren des ersten Hohlkörpers Z1 angeordneten Feder F, wie oben angegeben, abgefedert. Die Federkraft der sich stauchenden Feder F wird über einen Kraftsensor KS erfasst. Überschreitet die Federkraft der sich stauchenden Feder F einen vorgebbaren Wert, so ist die Wirkverbindungselementeeinheit WVE derart ausgebildet, dass sich die bislang verbundene erste und zweite Befestigungseinheit BE1, BE2 trennen. In dem ersten Hohlkörper Z1 ist in dieser Ausführungsvariante eine weitere mechanische Sicherungseinheit MOE integriert. Diese mechanische Sicherungseinheit MOE ist derart ausgebildet, dass ab einer zurückgelegten Wegstecke des zweiten Hohlkörpers Z2 in dem ersten Hohlkörper Z1 ein Öffnungsmechanismus in der Wirkverbindungselementeeinheit WVE betätigt wird und die erste von der zweiten Befestigungseinheit BE1, BE2 getrennt wird. In einer weiteren Ausführungsvariante kann die erste Befestigungseinheit BE1 der Koppeleinheit KE entfallen und die Wirkverbindungselementeeinheit WVE direkt mit einer hierfür vorgesehenen korrespondierenden Andockstelle an der Röntgeneinheit R und/oder Positionierungseinheit P in Verbindung gebracht werden.

In Figur 2 ist schematisch eine Röntgenanlage abgebildet. An dieser Röntgenanlage ist eine Gerätekombination aus Ultraschalleinheit US und Röntgeneinheit R angeordnet. Die Ultraschalleinheit US ist mit einer Koppeleinheit KE an der Röntgeneinheit R befestigt. Die Röntgeneinheit R ist in dieser Ausführungsvariante über einen an Schienen S geführten Schlitten SL und einem am Schlitten SL verfahrbaren Teleskoparm T im Raum zu einem positionierten Objekt bzw. Patienten PT ausrichtbar. In dieser Ausführungsvariante ist die erste Befestigungseinheit BE1 der Koppeleinheit KE an dafür vorgesehene Andockstellen bzw. Koppelstellen an der Röntgeneinheit R befestigbar. Der Schlitten SL ist entlang der beispielsweise an einem an der Decke befestigten Schienensystem S verfahrbar. Der in seiner Länge variierbare Teleskoparm T ist quer zur Bewegungsrichtung des Schlittens SL verfahrbar. Am Ende des Teleskoparms T ist eine, eine Vielzahl von ansteuerbaren Achsen aufweisende ausrichtbare Einheit angeordnet. An dieser Einheit ist dann die Röntgeneinheit R befestigt. Abgebildet ist ein, beispielsweise auf einer Liege, platzierter Patient PT. Soll eine Ultraschalluntersuchung mit Ultraschallaufnahmen von einer Region eines Patienten PT angelegt werden, so wird die an einer Koppeleinheit KE befestigte Ultraschalleinheit US an dafür vorgesehene Andockstellen an der Röntgeneinheit R bzw. der Positioniereinheit P befestigt. Entsprechend einer durchzuführenden Ultraschalluntersuchung wird die Ultraschalleinheit US an einen bestimmten Bereich am Patienten PT platziert und die Ultraschallaufnahme durch ein Verkippen bzw. Verkanten oder Schwenken der Ultraschalleinheit US angelegt. Der Assistent oder Arzt bedient über ein Bedienelement die Positionierungseinheit P. Die von der Bedieneinheit ausgelösten Steuerfunktionen werden über eine hier nicht explizit dargestellten Steuereinheit in Steuerimpulse für einzelne elektronisch ansteuerbare Elektromotoren in der Positioniereinheit P umgesetzt um eine bestimmungsgemäße zielgenaue Ausrichtung der Ultraschalleinheit US zu bewirken. Gezeigt sind in der Figur 2 eine mögliche Anfangsposition bzw. eine anfängliche Ausrichtung der Ultraschalleinheit US und eine mögliche Endposition der Ultraschalleinheit US am Patienten PT. Zur Erreichung dieser Positionierung der Ultraschalleinheit US wird beispielsweise der Schlitten SL entsprechend der angedeuteten Bewegungsrichtung verfahren, während der ausgefahrene Teleskoparm T geringfügig ein und wieder ausgefahren und gleichzeitig die Röntgeneinheit R geschwenkt wird. Die Steuereinheit kann dazu derart ausgebildet sein, dass ein voreinstellbarer Druck der Ultraschalleinheit US auf die vorbestimmte Körperregion des Patienten PT konstant gehalten wird. Muss sich der Patient PT unvorhersehbar bewegen, so wird diese Bewegung durch das in die Koppeleinheit KE integrierte Sicherungssystem, beispielsweise bestehend aus einer Überwachung des Federdrucks und einer mechanischen Notauslösung, bis zu einer patientenindividuell voreinstellbaren Abweichung abgefedert. Wird der Druck der Ultraschalleinheit US auf den Patienten PT größer, so werden Auslösemechanismen in der Wirkverbindungselementeeinheit WVE aktiviert und die Koppeleinheit KE löst sich beispielsweise von der mit ihr verbundenen Andockstelle an der Positioniereinheit P oder Röntgeneinheit R. Die Ultraschalleinheit US kann während der Ultraschalluntersuchung, gesteuert vom Arzt, bei gleichbleibendem Druck auf den Patienten PT geschwenkt bzw. gekippt werden. Ändert sich der Anpressdruck der Ultraschalleinheit US über ein vom Arzt bestimmbaren Anpressdruck, so öffnet sich entweder die Wirkverbindungselementeeinheit WVE zwischen der ersten und der zweiten Befestigungseinheit BE1, BE2 oder die Steuereinheit steuert alle elektronischen Motoren der Positioniereinheit P derart an, dass zumindest einzelne Komponenten von der Positionierungseinheit P zurückgefahren werden oder die Gelenke jeweils freigegeben und diese in eine vorgegebene oder sämtliche Richtungen annähernd Widerstandslos auslenkbar sind. In einer weiteren Ausgestaltung der Koppeleinheit KE kann vorgesehen werden, dass bei einem erhöhten Druck der Ultraschalleinheit US auf den Patienten PT der zweite Hohlzylinder Z2 gänzlich in den ersten Hohlzylinder Z1 einfahrbar ist. Der zweite Hohlzylinder ist dazu als Gleitelement GE ausgebildet. Des Weiteren könnte die erste Befestigungseinheit BE1 derart ausgebildet sein, dass zusätzlich die zweite Befestigungseinheit BE2 von dieser aufgenommen wird. Eine Bedieneinheit kann in einer weiteren Ausführungsvariante mit Steuerfunktionen für die Ultraschalleinheit US ausgebildet sein, dass mit dieser selbständig ein geradliniges Scannen, ein Kippen oder ein Schwenken durchführbar ist. Eine Führung der Ultraschalleinheit US über eine vorgegebene Trajektorie ist ebenfalls möglich.

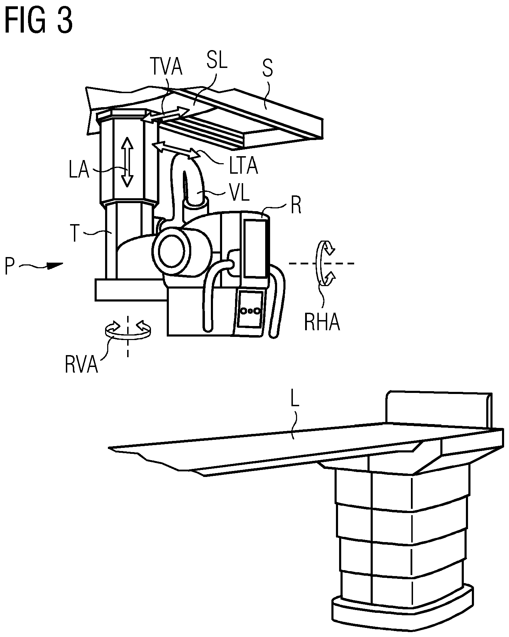

In der Figur 3 ist eine an einer Deckenschiene S befestigte Röntgeneinheit R abgebildet. Korrespondierend zu der über eine Deckenschiene S positionierbaren Röntgeneinheit R ist eine höhenverstellbare und schwenkbare Liege L, beispielsweise mit einer Detektoreinheit, positionierbar. Zur exakten Positionierung für eine Einzelaufnahme oder einer Vielzahl von Aufnahmen während einer Trajektorie der Röntgeneinheit R oder der an der Röntgeneinheit R befestigbaren Ultraschalleinheit US sind in dieser Figur die entsprechenden Achsen zur Ausrichtung des Röntgengerätes R bzw. der Ultraschalleinheit US angedeutet. Die Positionierung und Ausrichtung der Röntgeneinheit R bzw. der Ultraschalleinheit US wird mit einer Positioniereinheit P durchgeführt. In dieser Darstellung sind die zur Positionierung und Ausrichtung der Röntgeneinheit R bzw. Ultraschalleinheit US nötigen ansteuerbaren Achsen angedeutet. In dieser Ausführungsvariante ist am Ende des Teleskoparms T eine, eine Vielzahl von ansteuerbaren Achsen aufweisende ausrichtbare Einheit angeordnet. Mit dieser Einheit können eine Horizontalrotation RHA und beispielsweise eine Vertikalrotation um die Teleskopachse RVA durchgeführt werden. An dieser Einheit ist die Röntgeneinheit R befestigt. Die an dem Teleskoparm T angeordnete Röntgen- bzw. Ultraschalleinheit R, US ist mittels des Teleskoparms T, angedeutet durch den vertikalen Pfeil LA, in der Länge bzw. Höhe verstellbar. Der Teleskoparm T ist auf einem Schlitten SL sowohl longitudinal LTA als auch transversal TVA verfahrbar. Der Schlitten SL entlang der Schienen S an beliegen Stellen positionierbar.

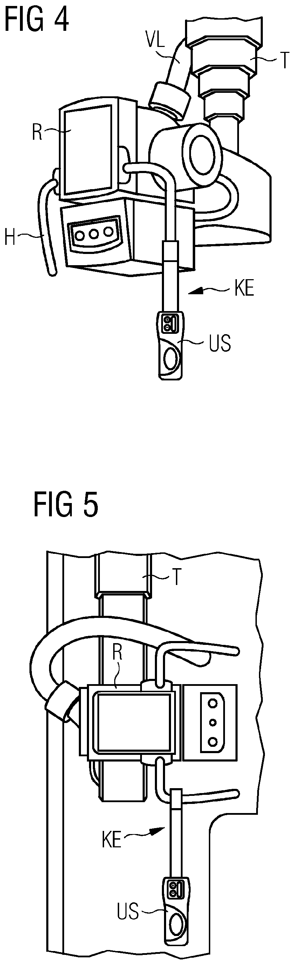

In Figur 4 ist ein Teilausschnitt der deckengehängten Röntgeneinheit R abgebildet. An dieser Röntgeneinheit R ist zu dessen manuellen oder semimanuellen Führung bzw. Ausrichtung ein Führungselement H vorgesehen. Dieses Führungselement H kann zu beiden Seiten der Röntgeneinheit R angeordnet sein. Eine an eine Ultraschalleinheit US angebrachte Koppelelement KE kann an dessen Ende derart ausgebildet sein, das mit diesem ein Kraftschluss an Elementen, beispielsweise einem Führungselement H, der Röntgeneinheit R und/oder an einer Andockstelle der unter Figur 3 beschriebenen Einheit am Ende des Teleskoparmes T der Positioniereinheit P ermöglicht wird. Neben dem Führungselement H als Andockelement können weitere Befestigungseinheiten für die Koppeleinheit KE der Ultraschalleinheit US an dem Chassis oder der Blendeneinheit der Röntgeneinheit R und/oder der Positioniereinheit P vorgesehen bzw. ausgebildet werden.

Figur 5 zeigt für das Koppelelement KE eine weitere Befestigungsvariante an dem Führungselement H der Röntgeneinheit R zur Führung der Ultraschalleinheit US.

### Bezugszeichenliste

- US: Ultraschalleinheit
- PT: Objekt, Patient
- L: Liegeeinheit
- SL: Schlitten
- S: Schiene
- T: Teleskoparm
- VL: Versorgungsleitungen
- R: Röntgeneinheit
- P: Positioniereinheit
- LA: Pfeil für höhenverstellbaren Teleskoparm
- TVA: Transversaltranslation
- LTA: Longitudinaltranslation
- RHA: Horizontalrotation
- RVA: Vertikalrotation um die Teleskopachse
- F: Feder
- KE: Koppelelement
- Z1: erster Hohlkörper
- Z2: zweiter Hohlkörper
- H: Führungselement
- KS: Kraftsensor
- GE: Gleitelement
- MOE: mechanisches Öffnungselement
- BE1: erstes Befestigungselement
- BE2: zweites Befestigungselement
- WVE: Wirkverbindungselementeeinheit

## Patentansprüche

1. Vorrichtung umfassend eine Röntgeneinheit (R) zum Erstellen von Röntgenaufnahmen von einem Objekt (PT), wobei die Röntgeneinheit (R) mit einer ausrichtbaren Positioniereinheit (P) auf das Objekt (PT) ausrichtbar ist, weiterhin ist eine Ultraschalleinheit (US) zum Erstellen von Ultraschallaufnahmen von dem Objekt (PT) vorgesehen, wobei die Ultraschalleinheit (US) mit einer Koppeleinheit (KE) an der Röntgeneinheit (R) befestigbar und auf das Objekt (PT) ausrichtbar ist,
**dadurch gekennzeichnet,**
**dass** bei einer Überschreitung eines vorgebbaren Anpressdrucks der Ultraschalleinheit (US) auf das zu untersuchende Objekt (PT) es zu einer Trennung der Koppeleinheit (KE) von der Röntgeneinheit (R) kommt.

2. Vorrichtung nach Patentanspruch 1,
**dadurch gekennzeichnet,**
**dass** die Koppeleinheit (KE) eine lösbare Befestigung für die an der Röntgeneinheit (R) und/oder Positioniereinheit (P) befestigte Ultraschalleinheit (US) aufweist.

3. Vorrichtung nach Patentanspruch 2,
**dadurch gekennzeichnet,**
**dass** die Koppeleinheit (KE) aus einer ersten und/oder einer zweiten Befestigungseinheit (BE1, BE2) gebildet ist, wobei mit der ersten oder zweiten Befestigungseinheit (BE1, BE2) eine lösbare Verbindung mit Elementen der Röntgen- und/oder Positioniereinheit (R, P) herstellbar ist.

4. Vorrichtung nach Patentanspruch 3,
**dadurch gekennzeichnet,**
**dass** zwischen der ersten und zweiten Befestigungseinheit(BE1, BE2) eine lösbare Wirkverbindungselementeeinheit (WVE) angeordnet ist.

5. Vorrichtung nach einem der vorhergehenden Patentansprüche,
**dadurch gekennzeichnet,**
**dass** die erste und/oder zweite Befestigungseinheit (BE1, BE2) der Koppeleinheit (KE) Sensorelemente (KS, MOE) zur Überwachung des Anpressdruckes der Ultraschalleinheit (US) auf das zu untersuchendes Objekt (PT) aufweist.

6. Vorrichtung nach einem der vorhergehenden Patentansprüche,
**dadurch gekennzeichnet,**
**dass** zur Trennung der Koppeleinheit (KE) von der Röntgen- (R) bzw. Positioniereinheit (P) in der Koppeleinheit (KE) eine Wirkverbindungselementeeinheit (WVE) vorgesehen ist.

7. Vorrichtung nach einem der vorhergehenden Patentansprüche,
**dadurch gekennzeichnet, dass**
die Positioniereinheit (P) eine Steuereinheit aufweist, wobei mit dieser Steuerimpulse für die zu steuernden Elektromotoren an den Achsen (LA, TVA, LTA, RHA, RVA) der Positioniereinheit (P) erzeugbar sind.

8. Verfahren zur Röntgen- und/oder Ultraschalluntersuchung von einem Objekt (PT) mit einer Röntgeneinheit (R) sowie einer Ultraschalleinheit (US), wobei die Röntgeneinheit (R) mit einer ausrichtbaren Positioniereinheit (P) auf das Objekt (PT) ausrichtbar ist, wobei die Ultraschalleinheit (US) zum Erstellen von Ultraschallaufnahmen von dem Objekt (PT) vorgesehen ist und mit einer Koppeleinheit (KE) an der Röntgeneinheit (R) befestigbar und auf das Objekt (PT) ausrichtbar ist,
**dadurch gekennzeichnet,**
**dass** eine Trennung der Befestigung der Ultraschalleinheit (US) an der Röntgeneinheit (R) bei einem Überschreiten eines vorgebbaren Anpressdrucks der Ultraschalleinheit (US) auf das Objekt (PT) eingeleitet und ausgeführt wird.

## Claims

1. Device comprising an x-ray unit (R) for producing x-ray recordings of an object (PT), wherein the x-ray unit (R) can be aligned with the object (PT) using an alignable positioning unit (P), furthermore an ultrasound unit (US) is provided for producing ultrasound recordings of the object (PT), wherein the ultrasound unit (US) can be fastened with a coupling unit (KE) to the x-ray unit (R) and aligned with the object (PT),
**characterised in that**
when a predeterminable contact pressure of the ultrasound unit (US) on the object (PT) to be examined is exceeded, the coupling unit (KE) becomes separated from the x-ray unit (R).

2. Device according to claim 1,
**characterised in that**
the coupling unit (KE) has a detachable fastening for the ultrasound unit (US) fastened to the x-ray unit (R) and/or positioning unit (P).

3. Device according to claim 2,
**characterised in that**
the coupling unit (KE) is formed from a first and/or a second fastening unit (BE1, BE2), wherein a detachable connection with elements of the x-ray and/or positioning unit (R, P) can be produced with the first or second fastening unit (BE1, BE2) .

4. Device according to claim 3,
**characterised in that**
a detachable active connecting element unit (WVE) is arranged between the first and the second fastening unit (BE1, BE2).

5. Device according to one of the preceding claims,
**characterised in that**
the first and/or second fastening unit (BE1, BE2) of the coupling unit (KE) has sensor elements (KS, MOE) for monitoring the contact pressure of the ultrasound unit (US) on the object (PT) to be examined.

6. Device according to one of the preceding claims,
**characterised in that**
an active connecting element unit (WVE) is provided in order to separate the coupling unit (KE) from the x-ray (R) or positioning unit (P) in the coupling unit (KE).

7. Device according to one of the preceding claims,
**characterised in that**
the positioning unit (P) has a control unit, wherein with this control pulses for the electric motors to be controlled can be generated on the axes (LA, TVA, LTA, RHA, RVA) of the positioning unit (P).

8. Method for x-ray examination and/or ultrasound examination of an object (PT) with an x-ray unit (R) and an ultrasound unit (US), wherein
the x-ray unit (R) can be aligned with the object (PT) using an alignable positioning unit (P),
wherein the ultrasound unit (US) is provided for producing ultrasound recordings of the object (PT) and can be fastened with a coupling unit (KE) to the x-ray unit (R) and can be aligned with the object (PT),
**characterised in that**
a separation of the fastening of the ultrasound unit (US) to the x-ray unit (R) is introduced and carried out when a predeterminable contact pressure of the ultrasound unit (US) on the object (PT) is exceeded.

## Revendications

1. Installation comprenant une unité (R) à rayons X pour produire des enregistrements à rayons X d'un objet (PT), l'unité (R) à rayons X pouvant être dirigée sur l'objet (PT) par une unité (P) de mise en position orientable,
dans laquelle il est, en outre, prévu une unité (US) à ultrasons pour produire des enregistrements à ultrasons de l'objet (PT),
l'unité (US) à ultrasons pouvant être fixée à l'unité (R) à rayons X par une unité (KE) d'accouplement et pouvant être dirigée sur l'objet (PT),
**caractérisée**
**en ce que**, lorsqu'une pression d'application pouvant être donnée à l'avance de l'unité (US) à ultrasons sur l'objet (PT) à examiner est dépassée, il se produit une séparation de l'unité (KE) d'accouplement de l'unité (R) à rayons X.

2. Installation suivant la revendication 1,
**caractérisée**
**en ce que** l'unité (KE) d'accouplement a une fixation pouvant être défaite de l'unité (US) à ultrasons fixée à l'unité (R) à rayons X et/ou à l'unité (P) de mise en position.

3. Installation suivant la revendication 2,
**caractérisée**
**en ce que** l'unité (KE) d'accouplement est formée d'une première et/ou d'une deuxième unité (BE1, BE2) de fixation, une liaison pouvant être défaite à des éléments de l'unité (R) à rayons X et/ou de l'unité (P) de mise en position pouvant être produite par la première ou par la deuxième unité (BE1, BE2) de fixation.

4. Installation suivant la revendication 3,
**caractérisée**
**en ce qu'**une unité (WVE) d'élément de liaison active pouvant être défaite est disposée entre la première et la deuxième unité (BE1, BE2) de fixation.

5. Installation suivant l'une des revendications précédentes,
**caractérisée**
**en ce que** la première et/ou la deuxième unité (BE1, BE2) de fixation de l'unité (KE) d'accouplement a des éléments (KS, MOE) de capteur pour le contrôle de la pression d'application de l'unité (US) à ultrasons sur l'objet (PT) à examiner.

6. Installation suivant l'une des revendications précédentes,
**caractérisée**
**en ce que**, pour séparer l'unité (KE) d'accouplement de l'unité (R) à rayons X ou de l'unité (P) de mise en position, il est prévu une unité (WVE) d'élément de liaison active dans l'unité (KE) d'accouplement.

7. Installation suivant l'une des revendications précédentes,
**caractérisée**
**en ce que** l'unité (P) de mise en position a une unité de commande, des impulsions pour les moteurs électriques à commander pouvant être produites par celle-ci sur les axes (LA, TVA, LTA, RHA, RVA) de l'unité (P) de mise en position.

8. Procédé d'examen par rayons X et/ou par ultrasons d'un objet (PT) par une unité (R) à rayons X ainsi que par une unité (US) à ultrasons, l'unité (R) à rayons X pouvant être dirigée sur l'objet (PT) par une unité (P) de mise en position orientable,
dans lequel l'unité (US) à ultrasons est prévue pour produire des enregistrements d'ultrasons de l'objet (PT) et peut être fixée à l'unité (R) à rayons X par une unité (KE) d'accouplement et être dirigée sur l'objet (PT),
**caractérisé**
**en ce que** l'on fait débuter et on réalise une séparation de la fixation de l'unité (US) à ultrasons à l'unité (R) à rayons X, si une pression d'application pouvant être donnée à l'avance de l'unité (US) à ultrasons sur l'objet (PT) est dépassée.
